(19) Europäisches Patentamt
European Patent Office
Office européen des brevets

(11) **EP 1 838 392 B1**

(12) **EUROPÄISCHE PATENTSCHRIFT**

(45) Veröffentlichungstag und Bekanntmachung des Hinweises auf die Patenterteilung:
**30.03.2011 Patentblatt 2011/13**

(51) Int Cl.:
*A61P 9/00* (2006.01)  *A61K 31/095* (2006.01)
*A61K 45/06* (2006.01)  *A61K 33/04* (2006.01)

(21) Anmeldenummer: **06700374.9**

(22) Anmeldetag: **02.01.2006**

(86) Internationale Anmeldenummer:
**PCT/EP2006/000007**

(87) Internationale Veröffentlichungsnummer:
**WO 2006/070022 (06.07.2006 Gazette 2006/27)**

(54) **SELEN-HALTIGE MEDIKAMENTE GEGEN ENDOTHELIALE GEFÄSSERKRANKUNGEN**

SELENIUM-CONTAINING MEDICAMENTS FOR THE PREVENTION OR TREATMENT OF ENDOTHELIAL VASCULAR DISEASES

MEDICAMENTS CONTENANT DU SELENIUM POUR PREVENIR OU TRAITER DES AFFECTIONS VASCULAIRES ENDOTHELIALES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **31.12.2004 DE 102004063638**

(43) Veröffentlichungstag der Anmeldung:
**03.10.2007 Patentblatt 2007/40**

(73) Patentinhaber: **Biosyn Arzneimittel GmbH**
**70734 Fellbach (DE)**

(72) Erfinder: **STIEFEL, Thomas**
**70734 Fellbach (DE)**

(74) Vertreter: **Grünecker, Kinkeldey,**
**Stockmair & Schwanhäusser**
**Anwaltssozietät**
**Leopoldstrasse 4**
**80802 München (DE)**

(56) Entgegenhaltungen:
EP-A- 1 479 390     WO-A-95/29684
WO-A-2005/023274    WO-A-2006/013602
WO-A-2006/018294    WO-A2-02/067853
WO-A2-2004/110338   DE-A1- 4 335 441
US-A1- 2002 012 715 US-A1- 2002 182 585

• TANGUY S ET AL: "PREISCHEMIC SELENIUM STATUS AS A MAJOR DETERMINANT OF MYOCARDIAL INFARCT SIZE IN VIVO IN RATS" ANTIOXIDANTS AND REDOX SIGNALING, MARY ANN LIEBERT, LARCHMONT, NY, US, Bd. 6, Nr. 4, 2004, Seiten 792-796, XP008035261 ISSN: 1523-0864
• D'ALESSIO, PATRIZIA ET AL: "Pharmacological modulation of alteration of endothelial cytoskeleton induced by hydrogen peroxide and TNF-.alpha." COMPTES RENDUS DES SEANCES DE LA SOCIETE DE BIOLOGIE ET DE SES FILIALES , 190(2-3), 289-297 CODEN: CRSBAW; ISSN: 0037-9026, 1996, XP008072703
• LU, JUNXUAN ET AL: "Antiangiogenic activity of selenium in cancer chemoprevention: Metabolite-specific effects" NUTRITION AND CANCER , 40(1), 64-73 CODEN: NUCADQ; ISSN: 0163-5581, 2001, XP008072715
• GUPTA, SNGH, SHARMA: "Neuroprotective effect of antioxidants on ischaemia..." PHARMACOLOGICAL RESEARCH, Bd. 48, Nr. 2, 2003, Seiten 209-215, XP008072698 India

**Beschreibung**

Die vorliegende Erfindung betrifft die Verwendung einer Selen-haltigen Verbindung zur Herstellung eines Medikaments zur Prophylaxe oder Therapie von endothelialen Gefäßerkrankungen, wo die flussabhängige Dilatation gesteigert wird.

**Hintergrund der Erfindung**

Zelluläre anti-oxidative Enzyme wie Glutathionperoxidase 1 und Superoxiddismutase spielen eine zentrale Rolle in der Kontrolle reaktiver Sauerstoffspezies. *In vitro* erhobene Daten sowie Studien in Tiermodellen deuten an, dass derartige Enzyme eine schützende Wirkung bei Arteriosklerose haben könnten. Die Relevanz dieser Enzyme bei Erkrankungen beim Menschen ist jedoch fraglich und umstritten.

Selen ist ein essentielles Spurenelement, das integraler Bestandteil verschiedener Proteine mit katalytischen Funktionen, wie z. B. Glutathionperoxidase, ist. In Tierstudien wird Selenmangel mit Kardiomyopathie und plötzlichem Kindstod sowie verringerten T-Zellzahlen und eingeschränkter Lymphozytenproliferation bzw. -reaktion in Verbindung gebracht.

Das vaskuläre Endothel hat eine Schlüsselrolle in der vaskulären Homeostase, die durch die Freisetzung autokriner und parakriner Substanzen bestimmt wird. Die Fehlfunktion von Endothelzellen ist ein systemischer Prozess und wahrscheinlich der Ausgangspunkt einer Arteriosklerose.

Erkrankungen des endothelialen Gefäßsystems zählen zu den wichtigsten Leiden in der Bevölkerung der Industriestaaten. Es gibt zahlreiche Ansätze für die Therapie dieser Erkrankungen, wobei jedoch immer noch der Bedarf nach alternativen bzw. verbesserten Behandlungsmethoden besteht. Selen-haltige Verbindungen waren schon als Antioxidationsmittel zur Behandlung von endothelialen Gefässerkrankungen bekannt: Siehe WO 200 5023274, DE 4335441.

Der vorliegenden Erfindung liegt das technische Problem zugrunde, neue Wege zur Prophylaxe und/oder Therapie von endothelialen Gefäßerkrankungen zur Verfügung zu stellen.

Dieses Problem wird durch die Verwendung einer Selen-haltigen Verbindung zur Prophylaxe bzw. Therapie von endothelialen Gefäßerkrankungen gelöst.

Es wurde in klinischen Studien überraschender Weise herausgefunden, dass die Verabreichung Selen-haltiger Verbindungen als Medikament zur Prophylaxe und/oder Therapie von endothelialen Gefäßerkrankungen in einem Säugetier, vorzugsweise einem Menschen, geeignet ist.

Es konnte insbesondere gezeigt werden, dass das erfindungsgemäße Mittel die flussabhängige Dilatation steigert sowie die oxidative Belastung als Folge einer endothelialen Gefäßerkrankung verringert.

Somit umfasst die vorliegende Erfindung die Verwendung Selen-haltiger Verbindungen zur Verabreichung an ein Säugetier, vorzugsweise einen Menschen, wobei die flussabhängige Dilatation gesteigert und optional die oxidative Belastung als Folge einer endothelialen Gefäßerkrankung verringert wird.

Des Weiteren betrifft die vorliegende Erfindung die Verwendung Selen-haltiger Verbindungen zur Steigerung der flussabhängigen Dilatation zur Behandlung endothelialer Gefäßerkrankungen, wie sie dem Facharzt für Gefäßerkrankungen üblicherweise bekannt sind, vorzugsweise zur Behandlung von Arteriosklerose, Herzinfarkt, und/oder Schlaganfall.

Als Selen-haltige Verbindungen zur Verwendung in der vorliegenden Erfindung können alle physiologisch verträglichen organischen und anorganischen Selen-haltigen Verbindungen zum Einsatz kommen, solange diese im Patienten physiologisch verträglich Selen insbesondere zur Aufnahme in Selen-haltige Enzyme bereitstellen. Vorzugsweise sind diese Verbindungen aus der Gruppe ausgewählt, die aus den unten angegebenen organischen Selenverbindungen und Selenit besteht.

Bevorzugte organische Selenverbindungen können wie folgt definiert werden:

$$R_1\text{-}(Se)_n\text{-}R_2,$$

wobei n eine ganze Zahl von 2 bis 8 bedeutet, und entweder

$R_1$ und $R_2$ gleich oder verschieden sind und geradkettige oder verzweigte Alkylreste mit 2 bis 36 Kohlenstoffatomen (bevorzugt mit 6 bis 12 Kohlenstoffatomen) bedeuten, wobei die Alkylreste am endständigen Kohlenstoffatom mit einer COOH-Gruppe substituiert sein können, oder

$R_1$ ein Wasserstoffatom und $R_2$ ein geradkettiger oder verzweigter Alkylrest mit 2 bis 36 Kohlenstoffatomen (bevorzugt mit 6 bis 12 Kohlenstoffatomen) bedeutet, wobei dieser Alkylrest am endständigen Kohlenstoffatom mit einer COOH-Gruppe substituiert sein kann.

Weiterhin sind Übergangselement und/oder Alkali- und/oder Erdalkaliselenit und/oder -selenat und/oder Selenige Säure bevorzugt.

In einer besonders bevorzugten Ausführungsform der vorliegenden Erfindung ist die Selen-haltige Verbindung Natriumselenit.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Kapseln, Pflaster, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen Selen-haltigen Verbindung enthalten die erfindungsgemäßen Arzneimittel üblicherweise weitere physiologisch verträgliche pharmazeutische Hilfsstoffe, die aus der Gruppe bevorzugt ausgewählt sind, die aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln besteht.

Die Auswahl der physiologisch verträglichen Hilfsstoffe sowie die einzusetzenden Mengen derselben hängt davon ab, ob das Arzneimittel oral, sublingual, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Selen-haltige Verbindungen in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäß verwendeten Selen-haltigen Verbindungen auch verzögert freisetzen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A. R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt.

Die an den Patienten zu verabreichende Menge der jeweiligen Selen-haltigen Verbindung kann variieren und ist beispielsweise vom Gewicht oder Alter des Patienten sowie von der Applikationsart, der Indikation und dem Schweregrad der Erkrankung abhängig. Üblicherweise werden mindestens eine Selen-haltige Verbindung in Mengen, die 100 bis 2000 $\mu$g Selen entspricht, pro Tag und Patient appliziert.

Vorzugsweise wird erfindungsgemäß mindestens eine Selen-haltige Verbindung in einer Dosierung von 100 bis 1000 $\mu$g, vorzugsweise 200 bis 600 $\mu$g, besonders bevorzugt 300 bis 500 $\mu$g pro Tag und Patient verabreicht.

Die Selen-haltige Verbindung wird vorzugsweise über einen Zeitraum von 1 bis 12 Monaten verabreicht.

Bevorzugt sind erfindungsgemäße Verwendungen zur Herstellung von Medikamenten zur oralen, vorzugsweise sublingualen oder parenteralen, vorzugsweise intravenösen Verabreichung der Selen-haltigen Verbindung.

Besonders bevorzugt sind erfindungsgemäße Verwendungen zur Herstellung von Medikamenten zur intravenösen Verabreichung.

Ganz besonders bevorzugt sind erfindungsgemäße Verwendungen zur Herstellung von Medikamenten zur oralen, vorzugsweise sublingualer Verabreichung, bei denen die Selen-haltige Verbindung bereits in der Mundschleimhaut resorbiert wird.

In einer weiteren Ausführungsform können Selen-haltige Verbindung(en) zusammen mit kardiologisch relevanten Wirkstoffen, vorzugsweise Nitroverbindungen, ß-Rezeptorblockern, Calciumantagonisten, Acetylsalicylsäure, Statinen oder Cholesterinsenkern, zur Herstellung eines Kombinationsmedikaments zur Behandlung von endothelialen Gefäßerkrankungen sowie weiteren kardiovaskulären Erkrankungen verwendet werden. Die Wirkungen der Arzneimittelkombinationen können synergistisch oder akkumulativ sein. Der Selen-haltige Wirkstoff und der weitere Wirkstoff können gleichzeitig oder getrennt verabreicht werden.

**Beschreibung der Figuren**

Fig. 1    zeigt grafisch die Veränderung des GPx-Spiegels im Blut einzelner Probanden über den Verlauf einer vierwöchigen Substitutionstherapie mit 200 oder 500 $\mu$g Se/Tag.

Fig. 2    zeigt grafisch die Veränderung des Selen-Spiegels im Blut einzelner Probanden über den Verlauf einer vierwöchigen Substitutionstherapie mit 200 oder 500 $\mu$g Se/Tag und Patient.

Fig. 3    zeigt grafisch die Veränderung des GPx-Spiegels im Blut einzelner Probanden über den Verlauf einer vierwöchigen Substitutionstherapie mit 500 $\mu$g Se/Tag und Patient.

Fig. 4    zeigt grafisch die Veränderung des GPx-Spiegels im Blut einer Probanden über den Verlauf einer vierwöchigen Substitutionstherapie mit 200 µg Se/Tag und Patient.

**Die folgenden Beispiele erläutern die Erfindung**

**Beispiel 1 Selentherapie als kardiovaskulärer Schutz**

*Allgemeine Angaben:*

17 Patienten im Alter von 50 bis 65 Jahren mit dokumentierter Koronarer Herzerkrankung (periphere arterielle Verschlusskrankheit mit Stadium II nach Fontaine) und einer dokumentierten flussabhängigen Dilatation von weniger als 5 % wurden randomisiert in zwei Gruppen: die erste Gruppe (n=7) erhielt über 4 Wochen täglich 200 µg Selen als Natriumselenit-haltige Trinklösung , die zweite Gruppe erhielt über 4 Wochen täglich 500 µg Selen als Natriumselenit-haltige Trinklösung. Vor Therapie und nach 4-wöchiger Therapie wurden funktionelle Tests durchgeführt und Blutproben genommen zur Bestimmung des Einflusses der unterschiedlichen Selengaben auf die Serum-Selenwerte, erythrozytäre Glutathionperoxidase (GPx-1) und andere für dieses Krankheitsbild relevante Laborparameter.

*Dosis:*

500 µg Selen als Natriumselenit n=10
200 µg Selen als Natriumselenit n=7

*Dauer der Therapie:*

4 Wochen

**Klinische Charakteristika der Studienpopulation**

**Tabelle 1**

| Variable | Selen 200 | Selen 500 | p-Wert |
|---|---|---|---|
| *Epidemiologische Charakteristika:* | | | |
| Alter in Jahren | 59.7±15.1 | 68.0±7.8 | 0.52 |
| Männliches Geschlecht (%) | 42.9 | 60 | 0.22 |
| *Klassische Risikofaktoren:* | | | $X^2$ |
| Diabetes | 2 (28.6%) | 4 (40%) | 0.39 |
| Hypertonie | 5 (71.4%) | 7 (70%) | 0.68 |
| Hyperlipidämie | 2 (28.6%) | 7 (70%) | 0.09 |
| Adipositas | 4 (57.1%) | 5 (50%) | 0.77 |
| Nikotinabusus | 6 (85.7%) | 8 (80%) | 0.88 |
| *Klinische Parameter:* | | | |
| MI in der Anamnese | 4 (57.1 %) | 8 (80%) | 0.31 |
| Mehrgefäßerkrankung | 6 (85.7%) | 7 (70%) | 0.81 |
| CCS II Minimum CCS I; Maximum CCS II | 4 (57.1%) | 6 (60%) | 0.91 |

(fortgesetzt)

| Variable | Selen 200 | Selen 500 | p-Wert |
|---|---|---|---|
| *Kardiale Medikation:* | | | |
| Betablocker | 4 (57.1%) | 7 (70%) | 0.59 |
| ACE-Hemmer | 1 (14.3%) | 7 (70%) | 0.025 |
| Statin | 3 (42.9%) | 6 (60%) | 0.49 |
| Mittelwert $\pm$ SD oder Anzahl (Prozent)<br>Mittelwertvergleich über ANOVA-Test, Vergleich kategorialer Variablen im Chi-Quadrat-Test nach Pearson | | | |

Ergebnisse:

In der folgenden Tabelle 2 wird der Einfluss von 200 µg Selen pro Tag und Proband (n=7) auf den Selengehalt im Serum und die erythrozytäre GPx-1 Aktivität im Vergleich zu anderen physiologischen Parametern gezeigt.

Tabelle 2

| Variable | Zeitpunkt 0 | Nach 4 Wochen Selen | Differenz | p-Wert |
|---|---|---|---|---|
| *Epidemiologische Charakteristika*: | | | | |
| Alter in Jahren | 59.7$\pm$15.1 | - | - | - |
| Männliches Geschlecht (%) | 42.9 | - | - | - |
| *Laborparameter*: | | | | |
| Selen im Serum (ng/ml) | 83.5$\pm$24.9 | 101.8$\pm$27.3 | 18.3 Faktor 1.2 | 0.18 |
| GPx (U/g Hb) | 31.2$\pm$8.6 | 34.8$\pm$9.8 | 3.6 Faktor 1.1 | 0.06 |
| *Leberwerte:* | | | | |
| GOT (U/l) | 22.0$\pm$4.0 | 24.7$\pm$8.0 | 1.7 | 0.3 |
| GPT (U/l) | 24.0$\pm$11.1 | 24.6$\pm$15.9 | 0.7 | 0.9 |
| *Nierenfunktion:* | | | | |
| Kreatinin (mg/dl) | 1.0$\pm$0.2 | 1.0$\pm$0.2 | 0 | 0.12 |
| *Inflammation*: | | | | |
| CRP (mg/l) | 7.8$\pm$7.6 | 6.2$\pm$6.9 | -1.6 | 0.05 |
| Kreatinkinase (CK) (U/l) | 122.5$\pm$79.0 | 148.1$\pm$10.3 | 25.6 | 0.09 |
| Alpha-Amylase (U/l) | 53.6$\pm$21.2 | 53.6$\pm$21.1 | 0 | 0.5 |
| *Blutfette*: | | | | |
| HDL-Cholesterin (mg/dl) | 61.4$\pm$13.11 | 61.0$\pm$11.9 | -0.4 | 0.8 |
| Lp (a) mg/dl | 27.3$\pm$10.8 | 39.9$\pm$33.5 | 12.6 | 0.8 |
| *Blutgerinnung*: | | | | |
| Quick (%) | 97.8$\pm$33.9 | 99.7$\pm$32.7 | 1.9 | 0.9 |
| Hb (g/dl) | 11.5$\pm$2.4 | 13.1$\pm$2.2 | 1.6 | 0.3 |
| Mittelwert $\pm$ SD oder Prozent<br>Mittelwertvergleich über ANOVA-Test, bzw. Wilcoxon-Test für verbundene Stichproben zum Konzentrationsvergleich Ausgangswert vs. 4-Wochen-Wert nach Substitution mit Natriumselenit. | | | | |

In Tabelle 3 wird der Einfluss von 500 µg Selen pro Tag und Proband (n=10) auf den Selengehalt im Serum und die erythrozytäre GPx-1 Aktivität im Vergleich zu anderen physiologischen Parametern gezeigt.

Tabelle 3

| Variable | Zeitpunkt 0 | Nach 4 Wochen Selen | Differenz | p-Wert |
|---|---|---|---|---|
| *Epidemiologische Charakteristika*: Alter in Jahren Männliches | 68.0±7.8 | - | - | - |
| Geschlecht (%) | 60 | - | - | - |
| *Laborparameter*: Selen im Serum (ng/ml) | 84.3±28.7 | 132.5±19.2 | 48.2 Faktor 1.6 | 0.01 |
| GPx (U/g Hb) | 27.9±8.1 | 34.9±8.5 | 7.0 Faktor 1.3 | 0.007 |
| *Leberwerte:* GOT (U/l) | 22.7±7.1 | 18.9±7.1 | -3.8 | 0.2 |
| GPT (U/l) | 23.0±10.7 | 21.5±7.2 | -1.5 | 0.5 |
| *Nierenfunktion*: Kreatinin (mg/dl) | 1.0±0.2 | 1.0±0.2 | 0 | 0.3 |
| *Inflammation:* CRP (mg/l) | 2.7±2.6 | 3.0±2.4 | 0.3 | 0.4 |
| Kreatinkinase (CK) (U/l) | 107.2±55.6 | 88.6±39.8 | -18.6 | 0.3 |
| Alpha-Amylase (U/l) | 64.0±36.6 | 70.7±17.8 | 6.7 | 0.5 |
| *Blutfette*: HDL-Cholesterin (mg/dl) | 60.2±18.4 | 56.9±18.7 | -3.3 | 0.6 |
| Lp (a) mg/dl | 71.4±61.8 | 94.9±62.3 | 23.5 | 0.04 |
| *Blutgerinnung:* Quick (%) | 97.6±41.6 | 105.1±29.1 | 7.5 | 0.9 |
| Hb (g/dl) | 13.0±1.6 | 13.9±0.3 | 0.9 | 0.2 |
| Mittelwert ± SD oder Prozent Mittelwertvergleich über ANOVA-Test, bzw. Wilcoxon-Test für verbundene Stichproben zum Konzentrationsvergleich Ausgangswert vs. 4-Wochen-Wert nach Substitution mit Natriumselenit. | | | | |

In Tabelle 4 wird tabellarisch die Veränderung des Selengehalts im Serum und erythroztäre GPx-Aktivität bei täglicher Selensubstitution der Probanden mit 200 oder 500 µg summarisch gezeigt.

Tabelle 4

| Variable | Gesamtgruppe Zeitpunkt 0 | Gesamtgruppe nach 4 Wochen | p-Wert |
|---|---|---|---|
| *Epidemiologische Charakteristika:* Alter in Jahren Männliches | 64.2±11.5 | - | |
| Geschlecht (%) | 55.6 | - | |
| *Laborparameter:* Selen im Serum (ng/ml) | 84.0±26.3 | 119.8±27.0 | 0.04 |

(fortgesetzt)

| Variable | Gesamtgruppe Zeitpunkt 0 | Gesamtgruppe nach 4 Wochen | p-Wert |
|---|---|---|---|
| GPx (U/g Hb) | 29.4±8.3 | 34.9±8.8 | 0.001 |
| | Differenz Selen 500μg (ΔZeitpunkt 0/4Wochen) | *Differenz Selen 200μg (ΔZeitpunkt 0/4Wochen)* | p-Wert* |
| Selen im Serum (ng/ml) | 18.3 | 48.2 | 0.07 |
| GPx (U/g Hb) | 7 | 3.6 | 0.38 |
| *Leberwerte:* | | | |
| GOT (U/l) | -3.8 | 1.7 | 0.19 |
| GPT (U/l) | -1.5 | 0.7 | 0.96 |
| *Nierenfunktion:* | | | |
| Kreatinin (mg/dl) | 0 | 0 | 0.81 |
| Inflammation | | | |
| CRP (mg/l) | 0.3 | -1.6 | 0.23 |
| Kreatinkinase (CK) (U/l) | -18.6 | 25.6 | 0.25 |
| Alpha-Amylase (U/l) | 0 | 0 | 0.6 |
| *Blutfette:* HDL-Cholesterin (mg/dl) | -3.3 | -0.4 | 0.4 |
| Lp (a) mg/dl | 23.5 | 12.6 | 0.2 |
| *Gerinnung:* | | | |
| Quick (%) | 7.5 | 1.9 | 0.5 |
| Hb (g/dl) | 0.9 | 1.6 | 0.96 |
| P-Wert für Ausgangs-Selen-Serumkonzentrationen bzw. Ausgangs-GPx-Konzentrationen im Vergleich der Subgruppen: P= 0.9, für GPx-Konzentrationen: P= 0.4 *P - Wert zur Analyse des Unterschiedes der Differenzen in den Subgruppen 200 und 500 μg Selen als Natriumselenit. | | | |

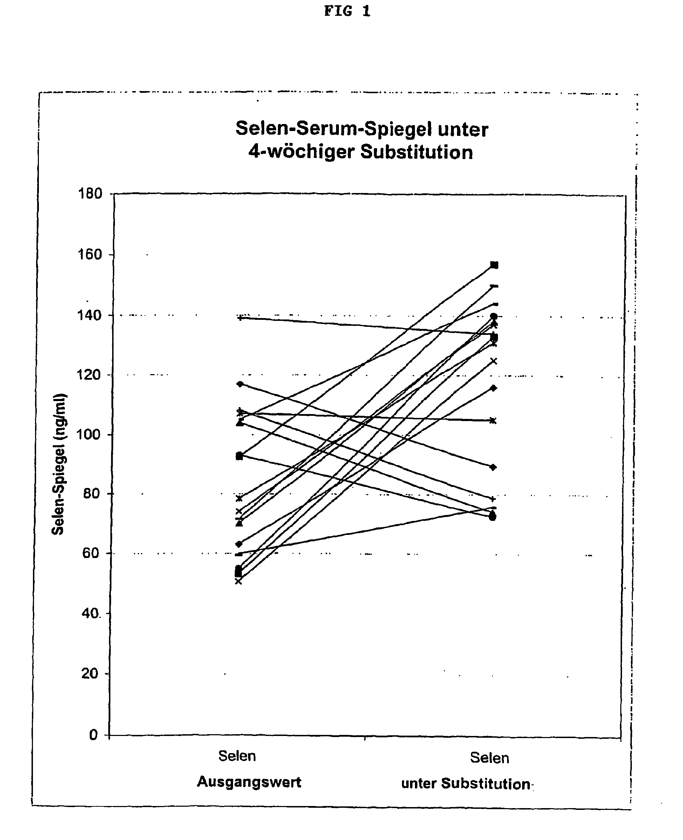

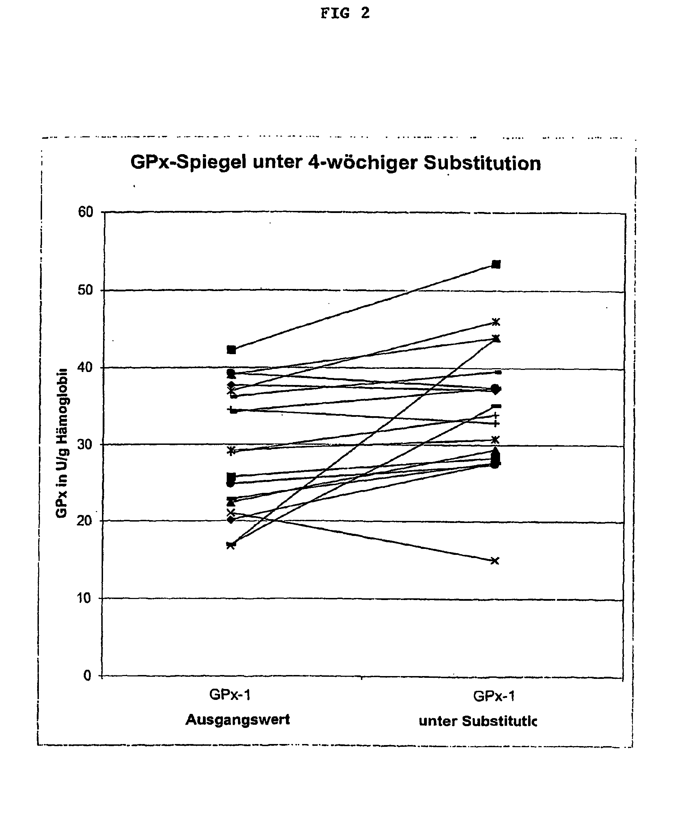

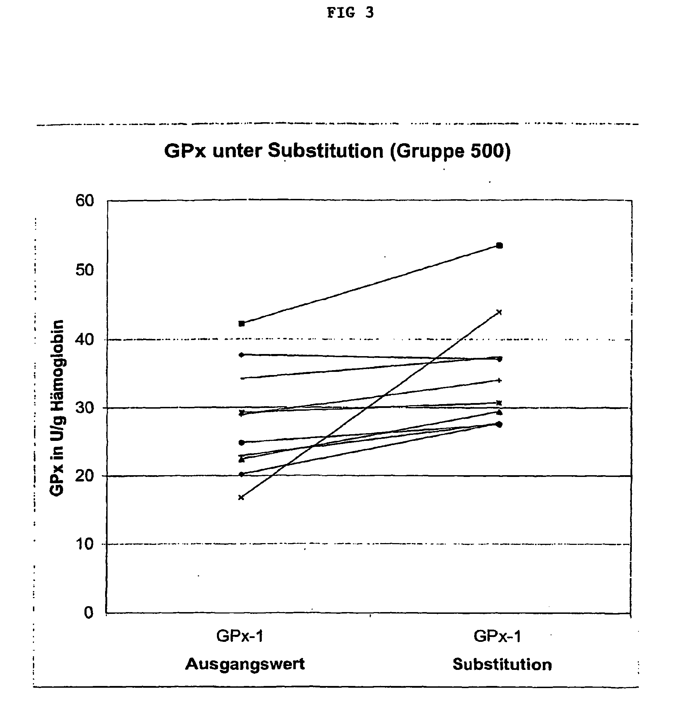

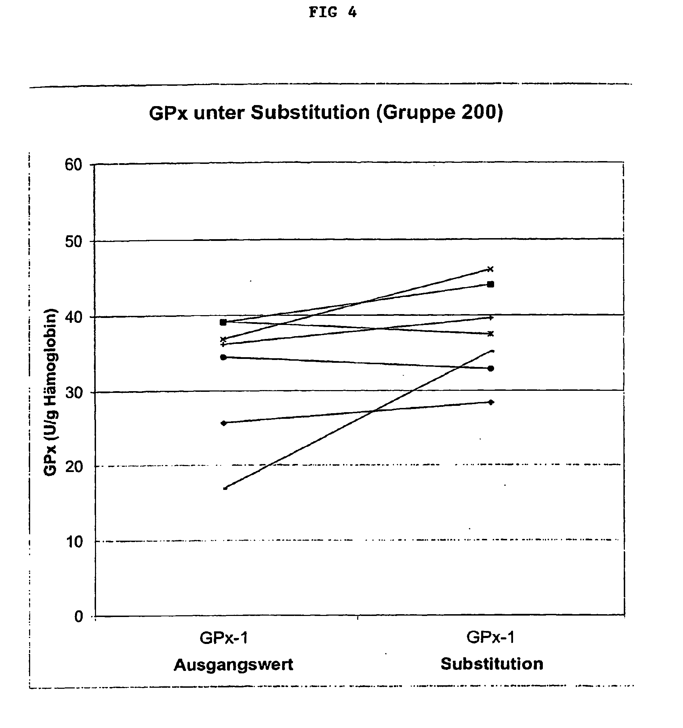

Diese Ergebnisse sind auch graphisch in den Figuren 1 bis 4 dargestellt.

Zusammenfassung der Ergebnisse

*Einfluss von Natriumselenit auf Kontrollparameter.*

Es wurden keine relevanten Veränderungen von Blutbild, Leberwerten oder hampflichtigen Substanzen beobachtet.

*Einfluss von Natriumselenit auf C-reaktives Protein:*

In der Gruppe mit 200 μg ist ein signifikanter Abfall von CRP nach 4 Wochen Therapie zu beobachten, in der Gruppe mit 500 μg Selen als Natriumselenit nicht. Das bedeutet, dass die höhere Dosis einen signifikanten antiinflammatorischen Effekt hat, der bei diesen Erkrankungen sehr wichtig ist.

*Einfluss von Natriumselenit auf Glutathionperoxidase:*

Natriumselenit hat einen Dosis-abhängigen Effekt auf die Aktivitätssteigerung der GPx-1. Während in der Gruppe mit 200 μg Natriumselenit ein nicht signifikanter Anstieg von 3.6 U/g Hb zu beobachten ist (31.2 auf 34.8 U/gHb), besteht in der 500 μg Therapiegruppe ein signifikanter Anstieg der GPx-1 um 7 U/gHb (27.9 auf 34.9 U/gHb, P=0.007).

*Zusammenfassend* besteht bei Patienten mit niedriger GPx-1 Aktivität ein relevanter und signifikanter Einfluss von Natriumselenit auf die Steigerung der GPx-1 Aktivität. Da niedrige GPx-1 Aktivität einen starken Prädiktor des kardiovaskulären Risikos darstellt und Natriumselenit die GPx-1 Aktivität steigert, kann die GPx-1 Aktivität als Indikator für die kardioprotektive Wirkung der Selentherapie herangezogen werden.

**Beispiel 2**

Bei einer weiteren Untersuchung wurde die fluß-mediierte Vasodilatation (FMD) bei Patienten mit hohen und niedrigen GPx-Werten gemessen.

| Geschlecht/Alter | GPx(U/gHb) | FMD |
|---|---|---|
| Weiblich 176 | 16,8 | 1,74 |
| Männlich / 65 | 20,2 | 1,33 |
| Männlich / 70 | 80,77 | 6,62 |
| Männlich / 74 | 76,66 | 5,81 |

Die Messwerte belegen, dass die Gabe von Selen sowohl die GPx-Aktivität, wie auch die FMD erhöht, wobei diese beiden Parameter bekanntermaßen den Verlauf endothelialer Gefäßerkrankungen maßgeblich bestimmen.

**Beispiel 3**

In dieser prospektiven Phase IV-Pilotstudie wurde bei Patienten mit peripherer arterieller Verschlusskrankheit im Stadium II nach Fontaine und nachgewiesenem Selenmangel die Wirkung von Selen (500 μg als Natriumselenit intravenös als Bolus) auf den Durchmesser der A. brachialis unter Ruhebedingungen sowie 5 und 30 Sekunden nach Lösen eines Staus (dreiminütiger Stau mittels Blutdruckmanschette am Oberarm) untersucht. Dazu wurde 30 Minuten nach einer Quantifizierung der flussvermittelten Vasodilatation (Kontrollphase) einmalig 500 μg Selen injiziert und die genannten Messgrößen erneut bestimmt.

Die Änderung im Gefäßdurchmesser der Probanden wurde wie folgt bestimmt Die Durchmesserbestimmungen der A. brachialis werden jeweils am rechten Arm mit Hilfe eines Acuson (Acuson 128 XP/4) Sonographiegerätes (Acuson, Mountain View, Kalifornien, USA) mit 7,5 MHz-Linearschallkopf durchgeführt: Sternitzky R, Blümel T, Jung F, Park J.-W., Schüler S. Endotheliale Dysfunktion nach Herztransplantation. VASA 2000; 26: 48.

Die Messungen erfolgen in standardisiertem Ablauf in der Methodik nach Mannion TC, Vita JA, Keaney JF, Benjamin EJ, Hunter L, Polak JF. Non-invasive assessment of brachial artery endothelial vasomotor function: the effect of cuff position on level of discomfort and vasomotor responses. Vascular medicine 1998; 3, 263-267.

1. Schritt: Ruhemessung

B-Bild-Sonographie der A. brachialis rechts. Die Einstellung erfolgt im Längsschnitt und Hinzuschalten des PW-Mode (Winkelkorrektur 60 Grad). Über die Gerätefunktion vaskuläre Berechnung erfolgt die Berechnung des Gefäßdurchmessers

2. Schritt: Postischämische Messung

Nach dreiminütiger Blutsperre (20 mmHg über dem systolischen Blutdruck) öffnen der Staumanschette und Messung des Durchmesser zu den Zeitpunkten 5 Sekunden und 30 Sekunden nach Öffnen des Staus.

3. Schritt: Berechnung der flussinduzierten Vasodilatation:

$$\text{FMD5 [\%]} = [(D5\text{-}DR)/DR] \times 100$$

$$\text{FMD30 [\%]} = [(D30\text{-}DR)/DR] \times 100$$

mit:

DR: Durchmesser der A.b. unter Ruhebedingungen
D5 : Durchmesser der A.b. 5 s nach Stauende
D30: Durchmesser der A.b. 30 s nach Stauende

4. Schritt: Nach 15 Minuten Ruhe erfolgt die Medikamentenapplikation mit einer Wiederholung der Schritte 1 bis 3.

Ergebnisse

Unter Ruhebedingungen beträgt der Durchmesser der A. brachialis bei den Patienten mit pAVK 4,14±0,44 mm. Unmittelbar nach der Injektion von Selen steigt der Durchmesser auf 4,65±0,45 mm also um im Mittel 0,51 mm oder 12,25% an (t-Test, unverbundene Stichproben, zweiseitige Fragestellung: p<0,0001). Damit zeigt sich in dieser klinischen Untersuchung unter Ruhebedingungen ein erheblicher Effekt von Selen auf den Durchmesser der A. brachialis.

Fünf Sekunden nach Lösen des Staus ist der Durchmesser der A. brachialis nach der Selengabe deutlich um 0,418 mm größer als ohne Selen (p<0,0001). Ähnlich ist es 30 Sekunden nach Lösen der Staus: hier ist der Mittelwert ebenfalls um 0,42 größer als vorher ohne Selen (p<0,0001).

Trotz der Vasodilatation unter Ruhebedingungen wird also die postischämische reaktive Hyperämie nicht negativ beeinflusst. Die durch Selen vermittelte Vasodilatation ist in der Phase der reaktiven Hyperämie genauso ausgeprägt wie unter Ruhebedingungen.

Die Durchmesser der A. brachialis waren vor beiden Prüfphasen identisch (p=0,945); eine Homogenität der Ausgangslage war vorhanden, ein carry-over-Effekt lag nicht vor.

Die Gefäßdurchmesser bzw. deren Veränderung sind ein Maß für die flussvermittelte Vasodilatation. Diese wiederum beschreibt das Ausmaß der peripheren arteriellen Verschlusskrankheit. Es konnte in dieser Studie der Nachweis geführt werden, dass Selen signifikant den Durchmesser der Gefäße im Ruhezustand und nach Stauung erweitert, damit die flussvermittelte Vasodilatation verbessert und damit zur Therapie von Patienten mit endothelialer Dysfunktion geeignet ist.

**Patentansprüche**

1. Verwendung einer Selen-haltigen Verbindung zur Herstellung eines Medikaments zur Steigerung der flussabhängigen Dilatation bei einer endothelialen Gefäßerkrankung.

2. Die Verwendung gemäß Anspruch 1, **dadurch gekennzeichnet, dass** das Medikament zur Verabreichung an ein Säugetier, vorzugsweise einen Menschen, formuliert ist.

3. Die Verwendung gemäß einem der Ansprüche 1 oder 2, **dadurch gekennzeichnet, dass** zusätzlich die oxidative Belastung als Folge einer endothelialen Gefäßerkrankung verringert wird.

4. Die Verwendung gemäß einem der Ansprüche 1 bis 3, **dadurch gekennzeichnet, dass** die endotheliale Gefäßerkrankung ausgewählt ist aus Arteriosklerose, Herzinfarkt, und Schlaganfall.

5. Die Verwendung gemäß einem der Ansprüche 1 bis 4, **dadurch gekennzeichnet, dass** die Selen-haltige Verbindung ausgewählt ist aus organischen Selenverbindungen und Selenit.

6. Die Verwendung nach Anspruch 5, **dadurch gekennzeichnet, dass** die Selen-haltige Verbindung Natriumselenit ist.

7. Die Verwendung gemäß einem der Ansprüche 1 bis 6, **dadurch gekennzeichnet, dass** die Selen-haltige Verbindung in einer Dosierung von 100 bis 2000 μg, vorzugsweise 200 bis 600 μg, besonders bevorzugt 300 bis 500 μg Se/ Tag und Patient zu verabreichen ist.

8. Die Verwendung gemäß einem der Ansprüche 1 bis 7, **dadurch gekennzeichnet, dass** das die Selen-haltige Verbindung oral, vorzugsweise sublingual, oder parenteral, vorzugsweise intravenös, zu verabreichen ist.

9. Die Verwendung nach Anspruch 7, **dadurch gekennzeichnet, dass** die Selen-haltige Verbindung intravenös zu verabreichen ist .

10. Die Verwendung nach Anspruch 7, **dadurch gekennzeichnet, dass** die Selen-haltige Verbindung oral, vorzugsweise sublingual, zu verabreichen ist , so dass es bereits durch die Mundschleimhaut resorbiert wird.

11. Die Verwendung gemäß einem der Ansprüche 1 bis 10, **dadurch gekennzeichnet, dass** die Selen-haltige Verbindung zusammen mit kardiologischen Arzneimitteln, vorzugsweise Nitroverbindungen, beta-Rezeptorenblockern, Calciumantagonisten, Acetylsalicylsäure, Statinen oder Cholesterinsenkern zu verabreichen ist .

12. Eine Selen-haltige Verbindung zur Verwendung in der Steigerung der flussabhängigen Dilatation bei einer endothelialen Gefäßerkrankung.

13. Die Selen-haltige Verbindung zur Verwendung gemäß Anspruch 12, **dadurch gekennzeichnet, dass** die endotheliale Gefäßerkrankung ausgewählt ist aus Arteriosklerose, Herzinfarkt, und Schlaganfall.

14. Die Selen-haltige Verbindung zur Verwendung gemäß Anspruch 12 oder 13, **dadurch gekennzeichnet dass** die Selen-haltige Verbindung ausgewählt ist aus organischen Selenverbindungen und Selenit, vorzugsweise Natriumselenit.

15. Die Selen-haltige Verbindung zur Verwendung gemäß einem der Ansprüche 12 bis 14, **dadurch gekennzeichnet, dass**

(i) die Selen-haltige Verbindung in einer Dosierung von 100 bis 2000 $\mu$g, vorzugsweise 200 bis 600 $\mu$g, besonders bevorzugt 300 bis 500 $\mu$g Se/ Tag und Patient zu verabreichen ist; und/oder
(ii) die Selen-haltige Verbindung oral, vorzugsweise sublingual, oder parenteral, vorzugsweise intravenös, zu verabreichen ist.

**Claims**

1. Use of a selenium-containing compound to produce a medicament to increase flow-dependent dilation in an endothelial vascular disease.

2. Use as claimed in claim 1, **characterised in that** the medicament is formulated for administration to a mammal, preferably a person.

3. Use as claimed in any one of claims 1 or 2, **characterised in that** in addition the oxidative loading as a consequence of an endothelial vascular disease is reduced.

4. Use as claimed in any one of claims 1 to 3, **characterised in that** the endothelial vascular disease is selected from arteriosclerosis, heart attack and stroke.

5. Use as claimed in any one of claims 1 to 4, **characterised in that** the selenium-containing compound is selected from organic selenium compounds and selenite.

6. Use as claimed in claim 5, **characterised in that** the selenium-containing compound is sodium selenite.

7. Use as claimed in any one of claims 1 to 6, **characterised in that** the selenium-containing compound is to be administered at a dose of 100 to 2000 $\mu$g, preferably 200 to 600 $\mu$g, particularly preferably 300 to 500 $\mu$g Se per day and patient.

8. Use as claimed in any one of claims 1 to 7, **characterised in that** the selenium-containing compound is to be administered orally, preferably sublingually, or parenterally, preferably intravenously.

9. Use as claimed in claim 7, **characterised in that** the selenium-containing compound is to be administered intravenously.

10. Use as claimed in claim 7, **characterised in that** the selenium-containing compound is to be administered orally, preferably sublingually, so that it is already resorbed by the mucous membrane of the mouth.

11. Use as claimed in any one of claims 1 to 10, **characterised in that** the selenium-containing compound is to be administered together with cardiological medicinal drugs, preferably nitro compounds, beta-receptor blockers, calcium antagonists, acetylsalicylic acid, statins or cholesterol reducers.

12. Selenium-containing compound for increasing flow-dependent dilation for use in the treatment of an endothelial vascular disease.

13. Selenium-containing compound as claimed in claim 12, **characterised in that** the endothelial vascular disease is selected from arteriosclerosis, heart attack and stroke.

14. Selenium-containing compound as claimed in claim 12 or 13, **characterised in that** the selenium-containing compound is selected from organic selenium compounds and selenite, preferably sodium selenite.

15. Selenium-containing compound as claimed in any one of claims 12 to 14, **characterised in that**

(i) the selenium-containing compound is to be administered at a dose of 100 to 2000 $\mu$g, preferably 200 to 600 $\mu$g, particularly preferably 300 to 500 $\mu$g Se per day and patient;
and/or
(ii) the selenium-containing compound is to be administered orally, preferably sublingually, or parenterally, preferably intravenously.

**Revendications**

1. Utilisation d'un composé contenant du sélénium pour fabriquer un médicament pour augmenter la dilatation dépendante du flux dans le cas d'une maladie de l'endothélium vasculaire.

2. Utilisation selon la revendication 1, **caractérisée en ce que** l'on formule le médicament pour une administration à un mammifère, de préférence, à un être humain.

3. Utilisation selon l'une quelconque des revendications 1 ou 2, **caractérisée en ce que** l'on réduit en outre la charge oxydante apparaissant consécutivement à une maladie de l'endothélium vasculaire.

4. Utilisation selon l'une quelconque des revendications 1 à 3, **caractérisée en ce que** la maladie de l'endothélium vasculaire est choisie parmi l'artériosclérose, l'infarctus du myocarde et l'attaque cérébrale.

5. Utilisation selon l'une quelconque des revendications 1 à 4, **caractérisée en ce que** le composé contenant du sélénium est choisi parmi des composés de sélénium organiques et le sélénite.

6. Utilisation selon la revendication 5, **caractérisée en ce que** le composé contenant du sélénium est le sélénite de sodium.

7. Utilisation selon l'une quelconque des revendications 1 à 6, **caractérisée en ce que** le composé contenant du sélénium est à administrer selon une posologie de 100 à 2000 $\mu$g, de préférence de 200 à 600 $\mu$g, mieux encore de 300 à 500 $\mu$g de Se par jour et par patient.

8. Utilisation selon l'une quelconque des revendications 1 à 7, **caractérisée en ce que** le composé contenant du sélénium est à administrer par voie orale, de préférence par voie sublinguale, ou par voie parentérale, de préférence par voie intraveineuse.

9. Utilisation selon la revendication 7, **caractérisée en ce que** le composé contenant du sélénium est à administrer par voie intraveineuse.

10. Utilisation selon la revendication 7, **caractérisée en ce que** le composé contenant du sélénium est à administrer par voie orale, de préférence par voie sublinguale, de sorte qu'il soit déjà absorbé par la muqueuse de la bouche.

11. Utilisation selon l'une quelconque des revendications 1 à 10, **caractérisée en ce que** le composé contenant du sélénium est à administrer conjointement avec des médicaments cardiologiques, de préférence des composés à fonction nitro, des agents bloquant les récepteurs bêta, des antagonistes du calcium, l'acide acétylsalicylique, des statines ou des hypocholestérolémiants.

12. Composé contenant du sélénium pour augmenter la dilatation dépendante du flux, pour une utilisation dans le traitement d'une maladie de l'endothélium vasculaire.

13. Composé contenant du sélénium selon la revendication 12, **caractérisé en ce que** la maladie de l'endothélium vasculaire est choisie parmi l'artériosclérose, l'infarctus du myocarde et l'attaque cérébrale.

14. Composé contenant du sélénium selon la revendication 12 ou 13, **caractérisé en ce que** le composé contenant du sélénium est choisi parmi des composés de sélénium organiques et le sélénite, de préférence, le sélénite de sodium.

15. Composé contenant du sélénium selon l'une quelconque des revendications 12 à 14, **caractérisé en ce que**

(i) le composé contenant du sélénium est à administrer selon une posologie de 100 à 2000 $\mu$g, de préférence, de 200 à 600 $\mu$g, mieux encore, de 300 à 500 $\mu$g de Se par jour et par patient ; et/ou
(ii) le composé contenant du sélénium est à administrer par voie orale, de préférence par voie sublinguale, ou par voie parentérale, de préférence par voie intraveineuse.

FIG 1

Selen-Serum-Spiegel unter
4-wöchiger Substitution

EP 1 838 392 B1

FIG 2

GPx-Spiegel unter 4-wöchiger Substitution

14

FIG 3

## GPx unter Substitution (Gruppe 500)

FIG 4

## GPx unter Substitution (Gruppe 200)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005023274 A
- DE 4335441

**In der Beschreibung aufgeführte Nicht-Patentliteratur**

- Remington's Pharmaceutical Sciences. Mack Publishing Company, 1985
- **Sternitzky R ; Blümel T ; Jung F ; Park J.-W. ; Schüler S.** Endotheliale Dysfunktion nach Herztransplantation. *VASA,* 2000, vol. 26, 48
- **Mannion TC ; Vita JA ; Keaney JF ; Benjamin EJ ; Hunter L ; Polak JF.** Non-invasive assessment of brachial artery endothelial vasomotor function: the effect of cuff position on level of discomfort and vasomotor responses. *Vascular medicine,* 1998, vol. 3, 263-267